(19) **Europäisches Patentamt / European Patent Office / Office européen des brevets**

(11) **EP 0 999 799 B1**

(12) **EUROPEAN PATENT SPECIFICATION**

(45) Date of publication and mention
of the grant of the patent:
**06.06.2007 Bulletin 2007/23**

(51) Int Cl.:
*A61B 19/00* (2006.01)  *A61B 17/02* (2006.01)
*A61B 19/08* (2006.01)

(21) Application number: **97936150.8**

(22) Date of filing: **21.07.1997**

(86) International application number:
**PCT/US1997/012800**

(87) International publication number:
**WO 1999/003416 (28.01.1999 Gazette 1999/04)**

(54) **SURGICAL RETRACTOR LINER APPLIANCE**

AUSKLEIDUNG FÜR CHIRURGISCHE WUNDHALTERBESTECKE

GARNITURE POUR ECARTEUR CHIRURGICAL

(84) Designated Contracting States:
**AT BE CH DE DK ES FI FR GB GR IE IT LI LU MC
NL PT SE**

(43) Date of publication of application:
**17.05.2000 Bulletin 2000/20**

(73) Proprietor: **MEDICAL CREATIVE
TECHNOLOGIES, INC.
Colmar, PA 18915-9727 (US)**

(72) Inventor: **CROOK, Berwyn, M.
Yardley, PA 19067 (US)**

(74) Representative: **Hanson, William Bennett et al
Bromhead Johnson
19 Buckingham Street
London WC2N 6EF (GB)**

(56) References cited:
| | |
|---|---|
| FR-A- 484 539 | US-A- 1 196 250 |
| US-A- 3 332 417 | US-A- 3 397 692 |
| US-A- 3 416 520 | US-A- 5 366 478 |
| US-A- 5 524 644 | US-A- 5 649 550 |

**Description**

Field of the Invention

The present invention relates generally to improvements in surgical devices, and more particularly to an improved adjustable retractor, and a retractor with a liner and integral drape therefor for preventing contamination of incised cavity walls of various thicknesses during surgery.

Background of the Invention

The sides of an open incision, as well as matter such as body parts and fluids passing through the incision during surgery, are inherently susceptible to cross-contamination by infectious microorganisms or like matter. Therefore, extreme care is required to insure that any exposed fluids or tissues are completely isolated from each other.

Various designs have been proposed and utilized to prevent transmission of indigenous and exogenous contaminants to healthy viscera from infectious tissues or fluids. U.S. Patent 5,524,644 by Berwyn M. Crook, filed June 9, 1995, describes an incision liner and retractor device which can be installed in an incision, incrementally adjusted in place to form-fit a wide range of cavity wall thicknesses, and retract the sides of the incision apart for better access to the abdominal cavity. It employs a flexible impermeable liner of pliable plastic material with opposite ends terminating at inner and outer resilient O-rings. The inner O-ring is inserted in the cavity by squeezing it through the incision and allowing it to expand around the inner edge of the incision. The outer O-ring is then rolled down over the portion of the liner extending out of the incision until it is tight against the outer rim of the incision and the remaining portion is drawn taut and contiguous with the incision sides. The outer O-ring is generally oblong in cross-section to provide a positive gripping surface for the fingers to roll the outer O-ring more easily, especially when the liner or the surgeon's gloves are slippery.

In many instances, a surgical drape may be first placed over the patient's body before the incision liner and retractor device is installed. This combination further reduces the risk of cross-contamination between the open cavity and the skin around the incision, especially if an organ is brought outside the abdominal cavity to perform surgery on it. However, since the liner and drape are not integrally connected, there is no assurance that the drape may not slide from beneath the outer O-ring and leave the patient's skin exposed in a most vulnerable region immediately adjacent to the incision.

Some prior art surgical protectors address this problem to a limited degree. For instance, U.S. Patent 3,397,692 to Creager, Jr. et al. discloses an incised surgical device in which a resilient ring cemented around the rim of a central aperture in a drape is squeezed together and expanded in the cavity to grip the incised edge of the peritoneum. The drape is bunched together where it passes through the incision and then spreads out over the body surface in radially diminishing wrinkles. U.S. Patent 4,188,945 to Wenander similarly provides a surgical cloth with a semi-rigid thread hemmed in around a central aperture. A portion of the cloth around the aperture is gathered together and inserted in an incision, and then enlarged under the incision edge by increasing the length of thread around the aperture. Like the surgical device of Creager, Jr. et al., a wrinkled surface is created in the incision and around the operating site. Consequently, neither device provides a relatively smooth surface in the incision and around the wound where extracted viscera may be placed nor positive retraction of the sides of the incision. In addition, there is no means for preventing external portions of the drape from slipping in and out of the incision with movement of the surgeon's hand.

US-A-5,524,644 describes an adjustable surgical wound protector according to the preamble of claim 1.

Objects of the Invention

Accordingly, it is an object of the present invention to provide an improved surgical retractor liner and integral drape which prevent exposure to cross-contamination by infectious fluids and solids between an incised cavity and the skin around the incision.

Another object of the invention is to provide a surgical retractor liner and integral drape assembly which interfaces smoothly and contiguously with the sides of an incision and with the skin around the incision.

Still another object of the invention is to provide a retractor liner with integral drape which can be easily installed and adjusted in place to fit a wide range of cavity wall thicknesses.

A further object is to provide a retractor liner which will positively insulate an incision from exposure to indigenous and exogenous contaminants and positively retract the sides of the incision for a wider opening to the cavity.

A further object of the present invention to provide a relatively low cost surgical retractor liner of simplified design which can be easily installed in a wound and adjusted in place to form fit a wide range of cavity wall thicknesses for protection against harmful contaminants.

Summary of the Invention

The present invention provides an adjustable surgical wound protector according to claim 1. Preferred or optional features of the invention are defined in the dependent claims.

Brief Description of the Drawings

The foregoing and other objects, features, and advantages of the invention will become more apparent from the following description of the preferred embodiments when taken in conjunction with the accompanying drawings wherein:

FIG. 1 represents a perspective top view of one embodiment of a surgical retractor liner and integral drape device according to the invention;
FIG. 2 is a side view partially in cross-section of a portion of the embodiment of FIG. 1;
FIG. 3 is an enlargement in cross section of an upper portion of the embodiment shown in FIGs. 1 and 2;
FIG. 4 is a view in radial cross section of an alternate embodiment of an O-ring for use in the upper portion of the assembly shown in FIG. 3; and
FIG. 5A and 5B are schematic illustrations of the device in two stages of installation in an incision;
FIG. 6 is an isometric view of another embodiment of a retractor liner according to the invention, in a fully extended state and with portions cut away;
FIG. 7 is a view in longitudinal cross-section of an outer end of the retractor liner of FIG. 6;
FIG. 8A and 8B is a schematic representation in longitudinal cross-section of the retractor liner of FIG. 6 partially installed in a surgical wound; and

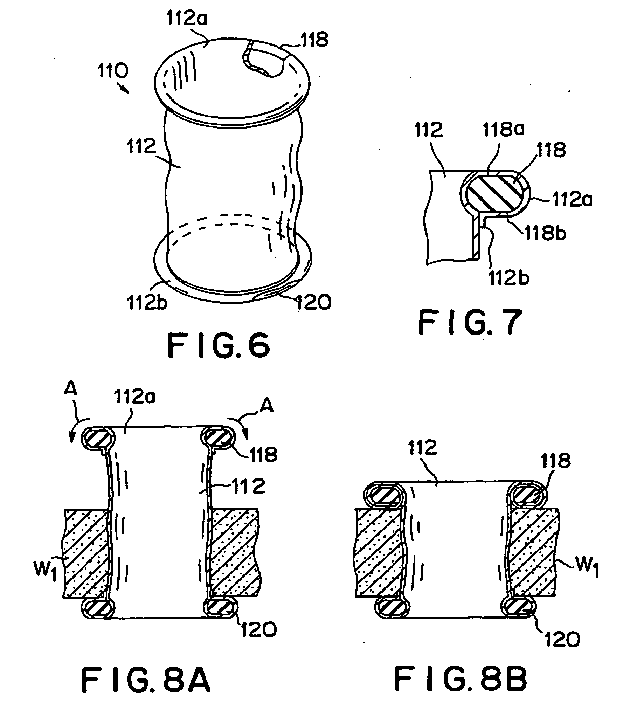

FIG. 8 schematically illustrates the retractor liner of FIG. 6 in two stages of installation in an incision.

Description of the Preferred Embodiments

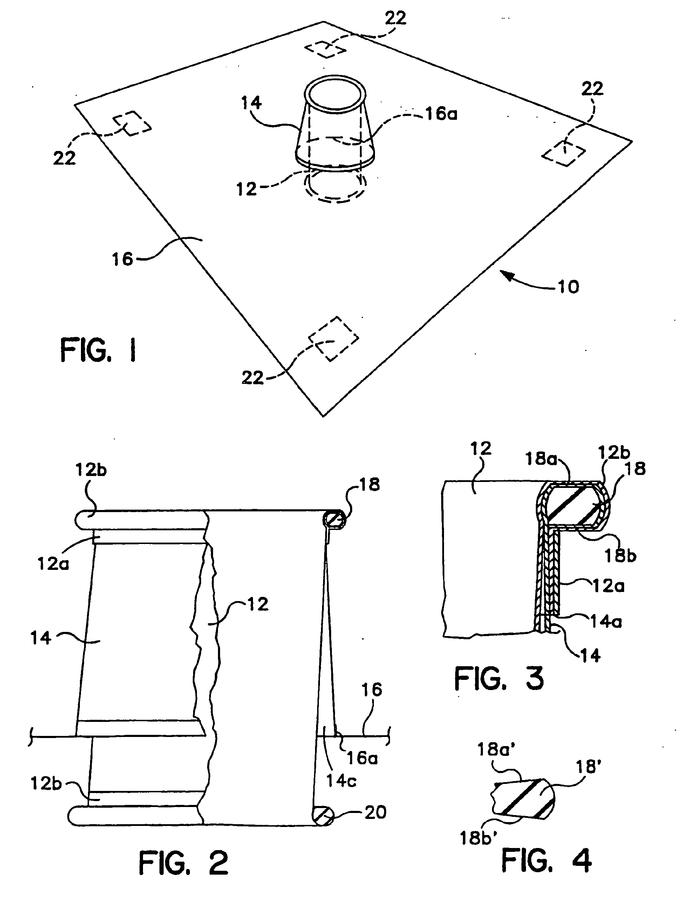

Referring now to the drawings wherein like reference characters designate like or corresponding parts throughout the several views, there is shown in FIG. 1 a surgical retractor liner and integral drape device 10 comprising an incision retractor liner 12 of uniform circumference along its length coaxially extending through a skirt 14 and a central aperture 16a in a drape 16.

As best seen in FIG. 2, retractor liner 12 and skirt 14 have adjacent upper ends which wrap around an outer O-ring 18 and overlap at annular edges 12a and 14a (FIG. 3) and seal to each other and to the outer side skirt 14. A lower end portion of retractor liner 12 wraps around an inner 0-ring 20 and overlaps at an annular edge 12b and seals to the outer side of retractor liner 12; whereas a lower end of skirt 14 is sealed around the perimeter of aperture 16a. Retractor liner 12 is essentially uniform in circumference along a central longitudinal axis defined by the extended length of the liner. Skirt 14 is coaxial with retractor liner 12 and tapers outwardly to aperture 16a.

outer O-ring 18 is generally oblate in cross-section with opposed upper and lower flat chordal sides 18a and 18b substantially normal to the extended length of retractor liner 12. Sides 18a and 18b are located equidistant from, and on opposite sides of, the centroid of the radial cross-section through O-ring 18. The oblate shape provides an over-center snap action when O-ring 18 is rolled about itself onto retractor liner 12 and skirt 14 for incrementally shortening the upper ends and for resisting unrolling after being shortened.

Inner O-ring 20 is entirely circular in cross-section, but may have a similar cross-section as O-ring 18 for incrementally shortening the lower end of retractor liner 12.

FIG. 4 illustrates in radial cross-section an alternate embodiment of an outer O-ring 18'. The upper and lower sides 18a' and 18b' taper outwardly from opposite sides of a plane normal to the extended length of liner 12 thereby allowing O-ring 18' to be turned with less resistance around its annular axis due to the lesser mass near the inner circumference O-ring 18'. This structure is particularly desirable for large diameter rings having relatively large diameter cross-sections.

The materials for making assembly 10 are selected to insure stability when installed. One preferred material for skirt 14 and drape 16 is a substantially inelastic heat-sealable 76 $\mu$m (3-mil) polyolefin plastic film, such as Saranex™ film 2050 by The Dow Chemical Company. Another preferred material for liner 12 and skirt 14 is a substantially elastic heat-sealable 51 $\mu$m (2-mil) polyurethane film, such as Dureflex® PT6100S by Deerfield Urethane, Inc.

The polyurethane material has been found to provide certain advantages not available with the Saranex™ polyolefin material. For instance, the elastic polyurethane material takes up any lengthwise adjustment which cannot be fully accommodated by the incremental adjustments made by rolling the O-ring 18'. In addition, the elastic polyurethane provides a better retraction function along the edges of the incised wound. This is believed due, at least in part, to the better wound edge margin gripping action resulting from the axial take-up of the elastic polyurethane.

The difference between the non-elastic polyolefin material and the elastic polyurethane may be seen from a test wherein five 1" x 3" specimens of each of these materials were stretch tested at room temperature (+75°F) and permanent deformation (deflection) calculated from its load vs. displacement curve. The average deflection of the polyolefin film was approximately 0.354 mm/mm (0.354 inch/inch), while the polyurethane specimen deflection averaged approximately 0.167 mm/mm (0.167 inch/inch).

O-rings 18 and 20 are preferably preformed of an elastomeric medical-grade polyurethane of sufficient hardness to retain the rings expanded in place around the inner and outer rims of the incision. The O-ring material must be compliant enough to allow the fingers to turn the outer O-ring 18 over 180° around its annular axis from the preformed configuration. They may be color-coded with different colors, such as white and blue, for easier recognition of the correct O-ring to be inserted in an incision.

Drape 16 may be adhered directly to the skin of the patient or to an underlying drape by an adhesive spread over the underside of the drape, or by adhesive patches 22 at selected locations on the underside of the drape. The size of drape 16 is selected to provide effective protection from exposure to infectious fluids and tissue in the vicinity of the incision.

The length of a fully extended retractor liner 12 is typically around 150mm to accommodate most wall thicknesses at the incision. An assortment of liner and O-ring diameters are provided to accommodate different lengths of incisions, and the personal preference of the surgeon. U.S. Patent No. 5,524,644, supra, discloses a table of liner and O-ring diameters available for different incision lengths, and its disclosure is incorporated by reference herein. The urethane O-rings are typically in the range of 50-90 Shore A durometers.

The diameter of an upper length of skirt 14, in a relaxed state before stretching around O-ring 18 and sealing it at edge 14a, corresponds substantially to the diameter of retractor liner 12. The remaining lower portion tapers outwardly to the diameter of aperture 16a which is slightly larger than the diameter of retractor liner 12 to allow clearance for retractor liner 12 to be stretched into contact with the outer rim of the incision. The length of skirt 14 must not be shorter than retractor liner 12 by an amount greater than the thickness of the wall at the incision. If the difference were greater, the drape will not adhere completely to the skin immediately adjacent to the incision. Of course, if the difference is less than the wall thickness, the lower end of skirt 14 will merely bunch up around the uninserted portion of retractor liner 12 and roll onto O-ring 18 but still provide a satisfactory seal. Typically, the thickness of abdominal walls ranges between 25mm and 75mm. Therefore, for an overall liner length of 150mm, an effective skirt length should not be shorter by more than 25mm, namely an overall length of 135mm.

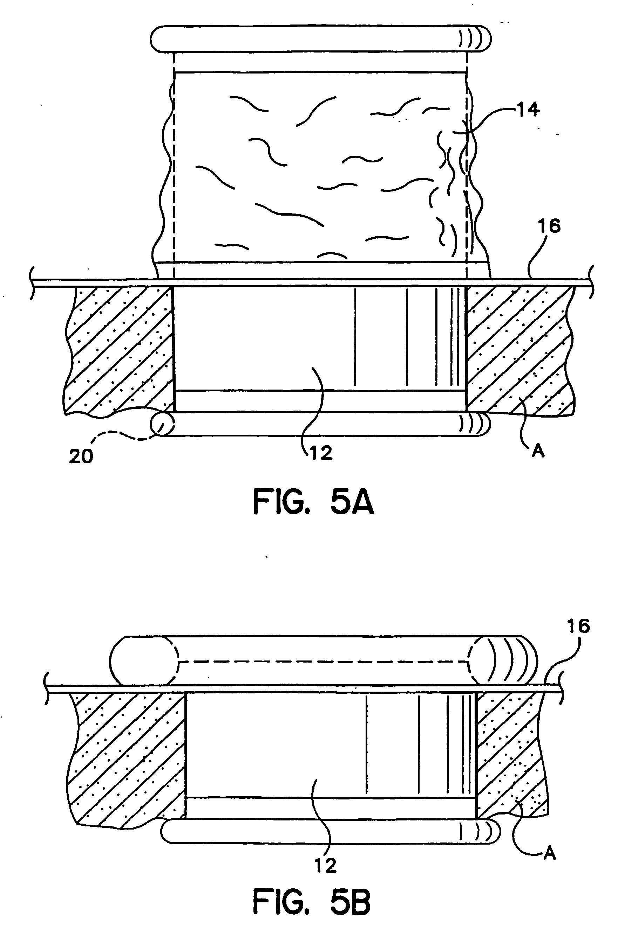

A typical installation of the retractor liner and integral drape assembly 10 is illustrated in two stages in FIGs. 5A and 5B. In FIG. 5a, retractor liner 12 is inserted into an incision in the abdominal wall A with the inner O-ring 20 expanded against the inner rim of the incision and drape 16 adhered to the skin, or to an underlying drape not shown. In this installation skirt 14 is shorter than retractor liner 12 by a difference slightly less than the thickness of the abdomen wall, thereby causing skirt 14 to bunch up around fully extended retractor liner 12. In FIG. 5B, the upper end of the assembly containing 0-ring 18 is rolled down over the outside of skirt 14, abuts the top of drape 16 with skirt 14 drawn taut against the incision, and retracts the sides of the incision to widen the opening. Drape 16 is thusly positively anchored against slipping out from under the rolled down portions of liner 12 and skirt 14.

Referring now to FIG. 6, an adjustable retractor device 110 includes a thin relatively elastic liner 112, uniform circumference along its length and impervious to solids and fluids containing bacteria and other harmful contaminants.

As best seen in FIG. 7, the upper end portion 112a of the liner 112 wraps around outer 0-ring 118 and terminates in an annular edge portion 112c sealed around the outer side of liner 112. At least one O-ring, such as the O-ring 118, is generally oblate in cross-section having opposed flat chordal side surfaces 118a and 118b which are transverse, i.e. substantially normal, to the liner central longitudinal axis defined by the extended length of liner 112, as shown in FIGs 6-8b. As shown, the chordal surfaces 118a and 118b are located equidistant from, and on opposite sides of, the centroid of the cross-section. The surfaces 118a and 118b provide surface means for purposes to be described.

Inner O-ring 120 is secured to lower end portion 112b in the same manner as O-ring 118, except the configuration in cross section is entirely circular. If desired, both O-rings may have the same cross-sectional shape as O-ring 118 to provide reversibility to the retractor liner device 110.

The oblate shape of the O-ring 118 provides stability in a plane perpendicular to the longitudinal axis of the liner 112 and provides an over center snap action when rolled about itself and the liner, thereby providing incremental shortening in predetermined increments and resistance to lengthening after shortening.

The materials and dimensions of adjustable surgical device 10 are selected to ensure stability when installed. A preferred elastic material suitable for liner 112 is a 2-mil polyurethane film, such as Dureflex® PT61005 supra. It is produced in seamless tubular form or by a flat sheet in a cylindrical form with the meeting margins along the side overlapped and sealed. A nominal liner length suitable for minimally invasive surgery is typically around 150 mm. Liner diameters will vary according to wound length as will be discussed.

Outer and inner O-rings 118 and 120 are preferably preformed of an elastomeric medical grade material of sufficient hardness to retain O-rings 118 and 120 expanded in place around the inner and outer edges of the wound.

Like O-ring 18, the material must be compliant enough to allow O-ring 118 to be turned by the fingers over 180 degrees around its annular axis from the preformed configuration. Urethane is therefore the preferred elastomeric material. When the O-rings are of different configurations, the O-rings are preferably color-coded with different colors, such as white and blue, for aiding in recognizing the correct end of the protector to be inserted in the wound.

The inside circumferences of O-rings 118 and 120 generally correspond to the outside circumference of liner 112. By way of example, a urethane O-ring 118 for use with a liner 110 mm (4.33 inches) in diameter has a diameter across the transverse cross section of about 7.94 mm (5/16 inch) with a distance between parallel flat sides 118a and 118b of approximately 6.10 mm (.240 inch). O-ring 120 has a diameter of its circular cross-section of about 7.94 mm (5/16 inch). Of course, the sizes of the O-rings and liners will vary according to wound size and wound wall thickness, and the personal preference of the surgeon will affect the choice of size for a particular surgical procedure.

The following table sets forth a preferred relation between incision length and liner and O-ring and liner diameters. It also sets forth the preferred cross-sectional diameters for each O-ring, it being understood that O-ring 18 has opposed flats and is, therefore, oblate and not circular in cross-section.

| Incision Length (mm) | Liner Diameter (mm) | O-Ring Cross Sectional Diameter (mm) |
|---|---|---|
| 10 | 30 | 5.15 |
| 20 | 30 | 5.15 |
| 30 | 60 | 7.13 |
| 40 | 60 | 7.13 |
| 50 | 80 | 7.52 |
| 60 | 80 | 7.52 |
| 70 | 110 | 7.92 |
| 80 | 110 | 7.92 |
| 90 | 110 | 7.94 |
| 100 | 130 | 9.53 |
| 110 | 130 | 9.53 |
| 120 | 150 | 11.11 |
| 130 | 150 | 11.11 |
| 140 | 170 | 12.70 |
| 150 | 170 | 12.70 |
| 160 | 190 | 14.29 |
| 170 | 190 | 14.29 |
| 180 | 210 | 15.88 |
| 190 | 210 | 15.88 |
| 200 | 230 | 15.88 |

The durometers of the O-rings set forth in the above table should be in a range of 50 to 90 Shore A. The preferred material is urethane, but silicone could be used with some loss of stability after installation and adjustment. The best stability is achieved by using a material having a high modulus of elasticity with a ring, as manufactured, having a minimum of residual stresses and strains. The size of the flats affects both gripability for adjustment and stability after adjustment, since the larger the size of flats for a given O-ring cross-sectional diameter, the less stability that exists. By way of example, a preferred flat width for an O-ring having a cross-sectional diameter of 7.94 mm (5/16 inch) is 6.10 mm (.240 inches). It is expected that with increasing diameters each flat width should increase proportionately based on a formula: $W = xD$ where $W$ is the width of the flat; $D$ is the diametrical cross-section of the O-ring; and $x$ is a constant equal to .85 for a urethane ring having a hardness within the ranges stated.

In using the adjustable surgical device in a minimally invasive abdominal surgical procedure, the abdomen is routinely prepared with antiseptics and dried; the site for the incision is traced on the abdomen and covered with a surgical drape; and a muscle-split is made at the site through the peritoneum. As illustrated in FIGS. 8A and 8B, retractor and liner 110 is placed in wound $W_1$ by squeezing inner O-ring 120 into a tight oblong shape and inserting it lengthwise through the incision and letting it expand inside the peritoneum around the inner edge of the wound. Outer end portion 112a is gripped by the thumb and fingers at flat sides 118a and 118b of outer ring 18 (FIG. 7) and turned outwardly, in opposite directions shown by arrows A, rolling liner 112 on the O-ring until it abuts the outer edge of the wound $W_1$ as shown in FIG. 8B. However, as a result of the elasticity of the liner material, any lengthwise adjustment not accommodated by the incremental adjustments causes the part of liner 112 in the wound between O-rings 118 and 120 to be thereby drawn into contiguous contact with the edge margins of wound $W_1$ and hence with the wound walls to provide a self-retaining protective barrier during surgery which is impervious to contaminating solids and fluids. If desired, the protector 10 can also be pre-adjusted prior to insertion, or partially pre-adjusted.

Some of the many advantages and novel features of the invention should now be readily apparent. For example, the invention provides an improved liner and drape device which prevents exposure between an incised cavity and the skin around the incision to cross-contamination by infectious fluids and tissue. The assembly is positively anchored in place around the operating site by the installed liner, smoothly interfaces against the sides of the incision and the surrounding skin, and retracts the sides of the incision for a wider opening. It can be easily installed in an incision and adjusted in place to fit a wide range of cavity wall thicknesses as well as provide positive insulation of an incision and surrounding skin from indigenous and exogenous contaminants.

A relatively simple and inexpensive surgical retractor liner is provided for protecting wounds from exposure to contamination. It can be quickly and easily installed in a wound and adjusted in place to form-fit a wide range of cavity wall thicknesses, and it stays in place after insertion. A fewer number of combinations of sizes of protectors are needed to accommodate a variety of incision sizes and cavity wall thicknesses.

Of course, it will be understood that various changes in the details, materials, steps and arrangement of parts, which have been herein described and illustrated in order to explain the nature of the invention, may be made by those skilled in the art within the scope of the invention as defined in the appended claims.

**Claims**

1. An incrementally adjustable surgical wound protector device comprising:

   an elongate tubular liner (12, 112) open at each of opposed ends thereof for inserting lengthwise in a wound, said liner (12, 112) being impervious to solid and fluid contaminants;
   an inner O-ring (20, 120) secured around one of said ends of said liner (12, 112), said inner O-ring (20, 120) having a preformed resilient configuration for overlapping the inner edge of the wound and for squeezing into an oblong shape insertable with a lengthwise portion of the liner (12, 112) adjacent to said inner O-ring (20, 120) in the wound and formed to expand against the inner edge of the wound; and
   an outer O-ring (18, 118) secured around the other of said opposed ends of said liner (12, 112), said outer O-ring (18, 118) having a non-circular radial cross-section with opposed surfaces enabling tactile gripping and rolling of said liner (12, 112) onto said outer O-ring (18, 118) and rolling the remaining lengthwise portion of the liner (12, 112) on itself about the outer O-ring (18, 118) to shorten the liner (12, 112) in predetermined increments and to resist subsequent lengthening when positioned to overlap the outer edge of the wound;

   whereby the liner length can be adjusted in increments before or after placement in the wound, **characterized by** said liner being made of a substantially elastic material, whereby the liner length is automatically adjustable lengthwise after placement.

2. An adjustable surgical wound protector according to claim 1, **characterized in that** said non-circular radial cross-section of said outer O-ring (18, 118) has a centroid and said opposed surfaces are on opposite sides of said centroid.

3. An adjustable surgical wound protector according to claim 1, **characterized in that** said opposed surfaces are substantially flat surfaces.

4. A surgical device (10) according to claim 1 for preventing cross-contamination during surgery of infectious fluids and solids between an incision and the external surface around the incision, further comprising:

   a continuous skirt (14) of flexible material having opposed first and second ends, said first end (14a) sealingly secured around said one liner end (12a) and coaxial with said liner (12) in their fully extended positions, said skirt (14) adapted to roll up with adjacent positions of said liner (12); and
   a drape (16) of flexible material having a central aperture (16a) sealingly secured around said second skirt end,

6

the length of said skirt (14) being sufficient for said drape (16) to adhere to the external surface immediately adjacent to the incision.

5. A device (10) according to claim 4 wherein said skirt (14) is shorter than said liner (12) in the extended positions.

6. A device (10) according to claim 4 wherein said length of said liner (12) exceeds the length of said skirt (14) by less than about 25mm.

7. A device (10) according to claim 4 wherein the length of said liner (12) exceeds the length of said skirt (14) by an amount in the range of about 25mm and 75mm.

8. A device (10) according to claim 4 wherein said liner (12) is of uniform circumference along the extended length, and said skirt (14) tapers outwardly along the extended length from said first skirt end (14a).

9. A device according to claim 4 wherein said substantially elastic material has a deformation of approximately 0.167 mm/mm (0.167 inch/inch).

10. A device (10) according to claim 4 wherein said drape (16) is made of a backing about 76 $\mu$m (3-mil) plastic sheet.

11. A device (10) according to claim 4 further comprising:

adhesive patches (22) disposed on one side of said drape (16) for adhering to a patient's skin or an underlying surgical drape.

12. A device (10) according to claim 4 wherein said outer O-ring (18) has substantially flat surfaces (18a, 18b) on opposite sides for enabling tactile gripping and rolling adjacent portions of said liner (12) and said skirt (14) onto said second resilient ring (18) in predetermined increments to shorten said liner (12) and to resist subsequent lengthening thereof.

13. A device (10) according to claim 12 wherein said outer O-ring surfaces (18a, 18b) are transverse to the extended length of said liner (12) and equidistant from the centroid of a radial cross-section thereof.

14. A device (10) according to claim 12 wherein said outer O-ring surfaces (18a, 18b) taper outwardly from a plane transverse to the extended length of said liner (12).

15. A surgical retractor device (10) according to claim 1, comprising:

a continuous skirt (14) of flexible material having opposed first and second ends, said first end (14a) sealingly secured around one of said liner ends (12a) and coaxial with said liner (12) in their fully extended positions.

16. A device (10) according to claim 15 wherein said skirt (14) is shorter than said liner (12) in the extended positions.

17. A device (10) according to claim 15 wherein said length of said liner (12) does not exceed the length of said skirt (14) by more than about 25mm.

18. A device (10) according to claim 15 wherein the length of said liner (12) exceeds the length of said skirt (14) by an amount in the range of about 25mm and 75mm.

19. A device (10) according to claim 15 wherein: said outer O-ring (18) includes opposed flat surfaces (18a, 18b) enabling tactile gripping and rolling of said skirt (14) and said liner (12) onto said outer O-ring (18).

20. A device (10) according to claim 19 wherein: said opposed surfaces (18a, 18b) lie in parallel planes perpendicular to a lengthwise extension of said liner (12) to provide said outer O-ring (12) with an oblate cross-section.

21. A device (10) according to claim 19 wherein said surfaces (18a', 18b') taper outwardly from a plane transverse to the extended length of said liner (12).

22. A surgical wound protector device (10) according to claim 1 incrementally adjustable lengthwise for use in surgery comprising:

a flexible skirt (14) open at opposite ends,
said outer ring (18) secured around one of said skirt ends (14a),
said outer ring (18) having an oblate transverse cross-section defined by a diametrically opposed pair of arcuate surfaces interconnected by an opposed pair of chordal surfaces (18a, 18b);
a flexible drape (16) having a central aperture (16a) secured around the other one of said skirt ends, the extended length of said skirt (14) being sufficient for said drape (16) to adhere the skin next to the incision; and
said inner (20) and outer (18) rings cooperating with said flexible liner (12) when installed in an incision both to protect the edges of the incision from contamination and to retract the edges from one another to facilitate surgery through the incision.

23. An adjustable surgical device (110) according to claim 1 wherein said elastic material has a defomation of approximately 0.167 mm/mm (0.167 inch/inch).

24. An adjustable surgical device (110) according to claim 1 wherein:

said opposed surfaces (118a, 118b) are disposed transverse to the length of said liner (112) in both an as-manufactured condition and an incrementally-adjusted condition.

25. An adjustable surgical device (110) according to claim 24 wherein:

said opposed surfaces (118a, 118b) are flat and are located in parallel planes equidistant from the centroid of said cross-section.

26. An adjustable surgical device (110) according to claim 25 wherein:

said parallel planes are perpendicular to the central longitudinal axis of the liner (112).

27. An adjustable surgical device (110) according to claim 25 wherein:

said liner (112) is a thin sheet of generally cylindrical form wrapped about said O-rings (118, 120) and secured thereto.

28. An adjustable surgical device (110) according to claim 1 wherein:

each of said O-rings (118, 120) is of urethane having a hardness in a range of about 50 to about 90 Shore A scale.

29. A lengthwise adjustable surgical device (110) according to claim 1 for use in surgery **characterized by**

said outer O-ring (118) having an oblate transverse cross-section defined by a diametrically opposed pair of arcuate surfaces interconnected by an opposed pair of chordal surfaces (118a, 118b),
said chordal surfaces (118a, 118b) extending outwardly from said liner (112) in a plane perpendicular to a central axis extending lengthwise of said liner (112),
said outer O-ring (118) being in a minimally strained stable condition when said chordal surfaces (118a, 118b) are lying in said plane,
said outer O-ring (118) is operable, when rolled 180° about its centroid, to roll said liner (112) about itself and thereby to adjust the length of the liner (112) in increments.

30. A lengthwise adjustable surgical device (110) according to claim 29 wherein at least said outer ring (118) is of urethane having a durometer in a range of about 50 to about 90 Shore A.

31. A lengthwise adjustable surgical device (110) according to claim 29 wherein the chordal width of each chordal surface (118a, 118b) is defined substantially by the formula:

$$W = xD,$$

wherein W is the width, x is a constant of .85, and D is the diametrical distance between said arcuate surfaces.

**Patentansprüche**

1. Inkremental verstellbare Schutzvorrichtung für eine Operationswunde, enthaltend:

   eine längliche rohrförmige Auskleidung (12, 112), die an ihren beiden entgegengesetzten Enden offen ist, um sie längs in eine Wunde einzusetzen, wobei die Auskleidung (12, 112) für feste und flüssige Verunreinigungen undurchlässig ist;
   einen inneren O-Ring (20, 120), der um eines der Enden der Auskleidung (12, 112) festgelegt ist, wobei der innere O-Ring (20, 120) eine vorgeformte nachgiebige Konfiguration zum Überlappen des inneren Randes der Wunde und zum Zusammenpressen in eine längliche Form aufweist, um ihn mit einem Längsabschnitt der dem inneren O-Ring (20, 120) benachbarten Auskleidung (12, 112) in die Wunde einzubringen, und der so geformt ist, dass er gegen den inneren Rand der Wunde expandiert; und
   einen äußeren O-Ring (18, 118), der um das andere der entgegengesetzten Enden der Auskleidung (12, 112) festgelegt ist, wobei der äußere O-Ring (18, 118) einen nicht-kreisförmigen radialen Querschnitt aufweist, bei dem die entgegengesetzten Oberflächen ein taktiles Ergreifen und das Aufrollen der Auskleidung (12, 112) auf den äußeren O-Ring (18, 118) und das Aufrollen des verbleibenden Längsabschnittes der Auskleidung (12, 112) auf sich selbst um den äußeren O-Ring (18, 118) ermöglichen, um die Auskleidung (12, 112) in vorbestimmten Inkrementen zu verkürzen und einer nachfolgenden Längenausdehnung standzuhalten, wenn sie zum Überlappen des äußeren Randbereiches der Wunde positioniert ist;

   wobei die Länge der Auskleidung vor oder nach der Anordnung in der Wunde in Inkrementen verstellt werden kann, **dadurch gekennzeichnet, dass** die Auskleidung aus einem im wesentlichen elastischen Material hergestellt ist, wobei die Länge der Auskleidung in Längsrichtung nach der Anordnung automatisch verstellbar ist.

2. Verstellbare Schutzvorrichtung für eine Operationswunde nach Anspruch 1, **dadurch gekennzeichnet, dass** der nicht-kreisförmige Querschnitt des äußeren O-Ringes (18, 118) einen Schwerpunkt aufweist und die entgegengesetzten Oberflächen auf entgegengesetzten Seiten des Schwerpunktes liegen.

3. Verstellbare Schutzvorrichtung für eine Operationswunde nach Anspruch 1, **dadurch gekennzeichnet, dass** die entgegengesetzten Oberflächen im wesentlichen flache Oberflächen sind.

4. Operationsvorrichtung (10) nach Anspruch 1 zur Verhinderung einer Kreuzkontamination infektiöser Flüssigkeiten und Feststoffe zwischen einem Schnitt und der äußeren Oberfläche um den Schnitt während einer Operation, die des weiteren enthält:

   einen durchgehenden Rock (14) aus flexiblem Material mit entgegengesetzten ersten und zweiten Enden, wobei das erste Ende (14a) abgedichtet um ein Ende (12a) der Auskleidung festgelegt ist und koaxial zu der Auskleidung (12) in ihren vollständig ausgestreckten Stellungen liegt, wobei der Rock (14) so angepasst ist, dass er sich mit benachbarten Stellen der Auskleidung (12) aufrollt; und
   ein Abdecktuch (16) aus flexiblem Material mit einer zentralen Öffnung (16a), die abgedichtet um das zweite Rockende festgelegt ist, wobei die Länge des Rockes (14) ausreicht, um das Abdecktuch (16) an der äußeren Oberfläche unmittelbar benachbart zu dem Schnitt anhaften zu lassen.

5. Vorrichtung (10) nach Anspruch 4, wobei der Rock (14) kürzer als die Auskleidung (12) in den ausgestreckten Stellungen ist.

6. Vorrichtung (10) nach Anspruch 4, wobei die Länge der Auskleidung (12) die Länge des Rockes (14) um weniger als 25 mm übersteigt.

7. Vorrichtung (10) nach Anspruch 4, wobei die Länge der Auskleidung (12) die Länge des Rockes (14) um einen Betrag im Bereich von etwa 25 mm bis 75 mm übersteigt.

8. Vorrichtung (10) nach Anspruch 4, wobei die Auskleidung (12) entlang ihrer ausgestreckten Länge einen gleichmäßigen Umfang aufweist und der Rock (14) entlang der ausgestreckten Länge vom ersten Rockende (14a) her nach außen aufgeweitet ist.

9. Vorrichtung nach Anspruch 4, wobei das im wesentlichen elastische Material eine Verformung von angenähert 0,167 mm/mm (0,167 inch/inch) aufweist.

10. Vorrichtung (10) nach Anspruch 4, wobei das Abdecktuch (16) aus einer etwa 76 μm (3-mil) Kunststofffolien-Verstärkung hergestellt ist.

11. Vorrichtung (10) nach Anspruch 4, weiterhin enthaltend:

    Klebepflaster (22), die auf einer Seite des Abdecktuches (16) angeordnet sind, um an der Patientenhaut oder einem darunter liegenden Operationsabdecktuch anzuhaften.

12. Vorrichtung (10) nach Anspruch 4, wobei der äußere O-Ring (18) im wesentlichen flache Oberflächen (18a, 18b) auf entgegengesetzten Seiten aufweist, um taktiles Ergreifen und das Aufrollen benachbarter Bereiche der Auskleidung (12) und des Rockes (14) auf den zweiten nachgiebigen Ring (18) in vorbestimmten Inkrementen zu ermöglichen, um die Auskleidung (12) zu verkürzen und einer nachfolgenden Längenzunahme derselben entgegenzuwirken.

13. Vorrichtung (10) nach Anspruch 12, wobei die Oberflächen (18a, 18b) des äußeren O-Ringes quer zur ausgestreckten Länge der Auskleidung (12) verlaufen und in gleichem Abstand vom Schwerpunkt des radialen Querschnittes desselben liegen.

14. Vorrichtung (10) nach Anspruch 12, wobei die Oberflächen (18a, 18b) des äußeren O-Ringes aus einer quer zur ausgestreckten Länge der Auskleidung (12) verlaufenden Ebene nach außen geneigt sind.

15. Chirurgischer Wundhalter (10) nach Anspruch 1, enthaltend:

    einen durchgehenden Rock (14) aus flexiblem Material mit entgegengesetzten ersten und zweiten Enden, wobei das erste Ende (14a) abdichtend um ein Ende (12a) der Auskleidung festgelegt ist und in ihren vollständig ausgestreckten Stellungen koaxial zu der Auskleidung (12) verläuft.

16. Vorrichtung (10) nach Anspruch 15, wobei der Rock (14) kürzer als die Auskleidung (12) in den ausgestreckten Stellungen ist.

17. Vorrichtung (10) nach Anspruch 15, wobei die Länge der Auskleidung (12) die Länge des Rockes (14) um nicht mehr als 25 mm übersteigt.

18. Vorrichtung (10) nach Anspruch 15, wobei die Länge der Auskleidung (12) die Länge des Rockes (14) um einen Betrag im Bereich von 25 mm bis 75 mm übersteigt.

19. Vorrichtung (10) nach Anspruch 15, wobei der äußere O-Ring (18) entgegengesetzte flache Oberflächen (18a, 18b) enthält, die ein taktiles Ergreifen und das Aufrollen des Rockes (14) und der Auskleidung (12) auf dem äußeren O-Ring (18) ermöglichen.

20. Vorrichtung (10) nach Anspruch 19, wobei die entgegengesetzten Oberflächen (18a, 18b) in parallelen Ebenen senkrecht zu einer Längsausrichtung der Auskleidung (12) liegen, um einen äußeren O-Ring (12) mit einem abgeflachten Querschnitt zu schaffen.

21. Vorrichtung (10) nach Anspruch 19, wobei die Oberflächen (18a', 18b') aus einer quer zur ausgestreckten Länge der Auskleidung (12) verlaufenden Ebene nach außen geneigt sind.

22. Chirurgische Wundschutzvorrichtung (10) nach Anspruch 1, die in Längsrichtung für den Einsatz bei Operationen inkremental einstellbar ist, umfassend:

    einen flexiblen Rock (14), der an entgegengesetzten Enden offen ist,
    den äußeren Ring (18), der um eines der Enden (14a) des Rockes festgelegt ist, wobei der äußere Ring (18) einen abgeflachten Querschnitt aufweist, der von einem diametral gegenüberliegenden Paar bogenförmiger Oberflächen gebildet wird, die durch ein gegenüberliegendes Paar von Sehnenoberflächen (18a, 18b) verbunden sind;

ein flexibles Abdecktuch (16) mit einer zentralen Öffnung (16a), welches um das andere der Rockenden festgelegt ist, wobei die ausgestreckte Länge des Rockes (14) ausreichend ist, um das Abdecktuch (16) an der Haut unmittelbar neben dem Schnitt anhaften zu lassen; und

wobei der innere und äußere Ring (20, 18) mit der flexiblen Auskleidung (12) zusammenwirken, wenn sie in einem Schnitt eingesetzt sind, sowohl zum Schutz der Ränder des Schnittes von einer Kontamination und um die Ränder voneinander zurückzuziehen, um die Operation durch den Schnitt zu erleichtern.

23. Verstellbare chirurgische Vorrichtung (110) nach Anspruch 1, wobei das elastische Material eine Verformung von annähernd 0,167 mm/mm (0,167 inch/inch) aufweist.

24. Verstellbare chirurgische Vorrichtung (110) nach Anspruch 1, wobei die gegenüberliegenden Oberflächen (118a, 118b) sowohl im Zustand wie hergestellt als auch in inkremental verstelltem Zustand quer zur Länge der Auskleidung (112) angeordnet sind.

25. Verstellbare chirurgische Vorrichtung (110) nach Anspruch 24, wobei die gegenüberliegenden Oberflächen (118a, 118b) flach ausgebildet sind und in parallelen Ebenen in gleichem Abstand vom Schwerpunkt des Querschnittes angeordnet sind.

26. Verstellbare chirurgische Vorrichtung (110) nach Anspruch 25, wobei die parallelen Ebenen senkrecht zur Mittellängsachse der Auskleidung (112) verlaufen.

27. Verstellbare chirurgische Vorrichtung (110) nach Anspruch 25, wobei die Auskleidung (112) eine dünne Folie von im wesentlichen zylindrischer Form ist, die um die O-Ringe (118, 120) gewunden und an diesen festgelegt ist.

28. Verstellbare chirurgische Vorrichtung (110) nach Anspruch 1, wobei jeder der O-Ringe (118, 120) aus einem Urethan mit einer Härte im Bereich von etwa 50 bis 90 Shore A besteht.

29. In Längsrichtung verstellbare chirurgische Vorrichtung (110) nach Anspruch 1 zur Verwendung bei Operationen, **dadurch gekennzeichnet, dass** der äußere O-Ring (118) einen abgeflachten Querschnitt aufweist, der von einem Paar diametral gegenüberliegender bogenförmiger Oberflächen definiert wird, die durch ein gegenüberliegendes Paar von Sehnenoberflächen (118a, 118b) verbunden sind,
wobei die Sehnenoberflächen (118a, 118b) sich von der Auskleidung (112) nach außen in einer senkrecht zur sich längs zur Mittelachse der Auskleidung (112) erstreckenden Ebene erstrecken,
wobei der äußere O-Ring (118) sich in einem minimal gedehnten stabilen Zustand befindet, wenn die Sehnenoberflächen (118a, 118b) in der Ebene liegen,
wobei der äußere O-Ring (118) durch Rollen um 180° um seinen Schwerpunkt dazu fähig ist, die Auskleidung (112) um sich selbst zu rollen und dabei die Länge der Auskleidung (112) in Inkrementen zu verstellen.

30. In Längsrichtung verstellbare chirurgische Vorrichtung (110) nach Anspruch 29, wobei zumindest der äußere Ring (118) aus einem Urethan hergestellt ist, welches ein Durometer im Bereich von 50 bis 90 Shore A aufweist.

31. In Längsrichtung verstellbare chirurgische Vorrichtung (110) nach Anspruch 29, wobei die Sehnenbreite jeder Sehnenoberfläche (118a, 118b) im wesentlichen durch die Formel definiert wird:

$$W = xD,$$

wobei W die Breite ist, x eine Konstante von 0,85 ist und D der diametrale Abstand zwischen den bogenförmigen Oberflächen ist.

**Revendications**

1. Dispositif protecteur de plaie chirurgicale ajustable par incréments comprenant :

un revêtement tubulaire allongé (12, 112) ouvert à chacune de ses extrémités opposées pour

insertion longitudinale dans une plaie, ledit revêtement (12, 112) étant imperméable aux contaminants solides et fluides ;

un joint torique interne (20, 120) fixé autour de l'une desdites extrémités dudit revêtement (12, 112), ledit joint torique interne (20, 120) ayant une configuration élastique préformée pour chevaucher le bord interne de la plaie et pour s'écraser en une forme oblongue insérable avec une partie longitudinale du revêtement (12, 112) adjacente au dit joint torique (20, 120) dans la plaie et formé pour s'étendre contre le bord interne de la plaie ; et

un joint torique externe (18, 118) fixé autour de l'autre desdites extrémités opposées dudit revêtement (12, 112), ledit joint torique externe (18, 118) ayant une section transversale radiale non circulaire avec des surfaces opposées permettant de prendre et de faire rouler tactilement ledit revêtement (12, 112) sur ledit joint torique externe (18, 118) et de faire rouler la partie longitudinale restante du revêtement (12, 112) sur elle-même autour du joint torique externe (18, 118) pour raccourcir le revêtement (12, 112) par incréments prédéterminés et pour résister à l'allongement subséquent après positionnement pour chevaucher le bord externe de la plaie ;

dans lequel la longueur du revêtement peut être ajustée par incréments avant ou après placement dans la plaie, **caractérisé en ce que** ledit revêtement est fabriqué en un matériau sensiblement élastique, la longueur du revêtement étant automatiquement ajustable en longueur après placement.

2. Protecteur de plaie chirurgicale ajustable selon la revendication 1, **caractérisé en ce que** ladite section transversale radiale non circulaire dudit joint torique (18, 118) a un centroïde et lesdites surfaces opposées sont sur des côtés opposés dudit centroïde.

3. Protecteur de plaie chirurgicale ajustable selon la revendication 1, **caractérisé en ce que** lesdites surfaces opposées sont des surfaces sensiblement plates.

4. Dispositif chirurgical (10) selon la revendication 1 pour éviter la contamination croisée pendant la chirurgie par des fluides et des solides infectieux entre une incision et la surface externe autour de l'incision, comprenant en outre :

une collerette continue (14) en matériau flexible ayant une première extrémité et une seconde extrémité opposées, ladite première extrémité (14a) étant fixée de manière étanche autour de ladite une extrémité de revêtement (12a) et étant coaxiale avec ledit revêtement (12) dans leurs positions entièrement étendues, ladite collerette (14) étant adaptée pour rouler avec des positions adjacentes dudit revêtement (12) ; et

un drap (16) en matériau flexible ayant une ouverture centrale (16a) fixée de manière étanche autour de ladite seconde extrémité de collerette, la longueur de ladite collerette (14) étant suffisante pour que ledit drap (16) adhère à la surface externe immédiatement adjacente à l'incision.

5. Dispositif (10) selon la revendication 4, dans lequel ladite collerette (14) est plus courte que ledit revêtement (12) dans les positions étendues.

6. Dispositif (10) selon la revendication 4, dans lequel ladite longueur dudit revêtement (12) dépasse la longueur de ladite collerette (14) de moins d'environ 25 mm.

7. Dispositif (10) selon la revendication 4, dans lequel la longueur dudit revêtement (12) dépasse la longueur de ladite collerette (14) d'une quantité dans la plage d'environ 25 mm à 75 mm.

8. Dispositif (10) selon la revendication 4, dans lequel ledit revêtement (12) a une circonférence uniforme le long de la longueur étendue, et ladite collerette (14) s'affine vers l'extérieur le long de la longueur étendue à partir de ladite première extrémité (14a) de collerette.

9. Dispositif selon la revendication 4, dans lequel ledit matériau sensiblement élastique a une déformation d'environ 0,167 mm/mm (0,167 pouce/pouce).

10. Dispositif (10) selon la revendication 4, dans lequel ledit drap (16) est fabriqué en un support en feuille de plastique d'environ 76 mm (3 mil).

11. Dispositif (10) selon la revendication 4, comprenant en outre :

des patches adhésifs (22) placés sur un côté dudit drap (16) pour adhérer à la peau d'un patient ou à un drap chirurgical sous-jacent.

12. Dispositif (10) selon la revendication 4, dans lequel ledit joint torique externe (18) a des surfaces sensiblement plates (18a, 18b) sur des côtés opposés pour permettre de prendre et de faire rouler tactilement des parties adjacentes dudit revêtement (12) et ladite collerette (14) sur ledit second anneau résilient (18) selon des incréments prédéterminés pour raccourcir ledit revêtement (12) et résister à son allongement subséquent.

13. Dispositif (10) selon la revendication 12, dans lequel lesdites surfaces de joint torique externe (18a, 18b) sont transversales à la longueur étendue dudit revêtement (12) et équidistantes du centroïde d'une section transversale radiale de celui-ci.

14. Dispositif (10) selon la revendication 12, dans lequel lesdites surfaces (18a, 18b) de joint torique externe s'affinent vers l'extérieur depuis un plan transversal à la longueur étendue dudit revêtement (12).

15. Dispositif d'écarteur chirurgical (10) selon la revendication 1, comprenant :

une collerette continue (14) en matériau flexible ayant une première extrémité et une seconde extrémité opposées, ladite première extrémité (14a) étant fixée de manière étanche autour de l'une desdites extrémités du revêtement (12a) et coaxiale au dit revêtement (12) dans leurs positions entièrement étendues.

16. Dispositif (10) selon la revendication 15, dans lequel ladite collerette (14) est plus courte que ledit revêtement (12) dans les positions étendues.

17. Dispositif (10) selon la revendication 15, dans lequel ladite longueur dudit revêtement (12) ne dépasse pas la longueur de ladite collerette (14) de plus d'environ 25 mm.

18. Dispositif (10) selon la revendication 15, dans lequel la longueur dudit revêtement (12) dépasse la longueur de ladite collerette (14) d'une quantité dans la plage d'environ 25 mm à 75 mm.

19. Dispositif (10) selon la revendication 15, dans lequel: ledit joint torique externe (18) inclut des surfaces plates opposées (18a, 18b) permettant de prendre et de faire rouler tactilement ladite collerette (14) et ledit revêtement (12) sur ledit joint torique externe (18).

20. Dispositif (10) selon la revendication 19, dans lequel: lesdites surfaces opposées (18a, 18b) reposent dans des plans parallèles, perpendiculaires à une extension longitudinale dudit revêtement (12), pour fournir au dit joint torique externe (12) une section transversale aplatie.

21. Dispositif (10) selon la revendication 19, dans lequel lesdites surfaces (18a', 18b') s'affinent vers l'extérieur à partir d'un plan transversal à la longueur étendue dudit revêtement (12).

22. Dispositif protecteur de plaie chirurgicale (10) selon la revendication 1, ajustable longitudinalement par incréments pour une utilisation en chirurgie, comprenant :

une collerette flexible (14) ouverte aux extrémités opposées,
ledit anneau externe (18) étant fixé autour d'une desdites extrémités (14a) de la collerette, ledit anneau externe (18) ayant une section transversale aplatie définie par une paire diamétralement opposée de surfaces arquées interconnectées par une paire opposée de surfaces chordales (18a, 18b) ;
un drap flexible (16) ayant une ouverture centrale (16a) fixée autour de l'autre desdites extrémités de collerette, la longueur étendue de ladite collerette (14) étant suffisante pour que ledit drap (16) adhère à la peau à côté de l'incision ; et
lesdits anneaux interne (20) et externe (18) coopérant avec ledit revêtement flexible (12) lorsqu'ils sont installés dans une incision à la fois pour protéger les bords de l'incision contre la contamination et pour écarter les bords l'un de l'autre pour faciliter la chirurgie à travers l'incision.

23. Dispositif chirurgical ajustable (110) selon la revendication 1, dans lequel ledit matériau élastique a une déformation d'environ 0,167 mm/mm (0,167 pouce/pouce).

24. Dispositif chirurgical ajustable (110) selon la revendication 1, dans lequel :

lesdites surfaces opposées (118a, 118b) sont disposées transversalement à la longueur dudit revêtement (112) aussi bien à l'état sortant de l'usine qu'à l'état ajusté par incréments.

25. Dispositif chirurgical ajustable (110) selon la revendication 24, dans lequel :

   lesdites surfaces opposées (118a, 118b) sont plates et sont situées dans des plans parallèles équidistants du centroïde de ladite section transversale.

26. Dispositif chirurgical ajustable (110) selon la revendication 25, dans lequel :

   lesdits plans parallèles sont perpendiculaires à l'axe longitudinal central du revêtement (112).

27. Dispositif chirurgical ajustable (110) selon la revendication 25, dans lequel:

   ledit revêtement (112) est une mince feuille de forme généralement cylindrique enroulée autour desdits joints toriques (118, 120) et fixée à ceux-ci.

28. Dispositif chirurgical ajustable (110) selon la revendication 1, dans lequel:

   chacun desdits joints toriques (118, 120) est en uréthane ayant une dureté dans une plage d'environ 50 à environ 90 Shore A.

29. Dispositif chirurgical ajustable en longueur (110) selon la revendication 1, à utiliser en chirurgie, **caractérisé par** ledit joint torique externe (118) ayant une section transversale aplatie définie par une paire diamétralement opposée de surfaces arquées interconnectées par une paire opposée de surfaces chordales (118a, 118b), lesdites surfaces chordales (118a, 118b) s'étendant vers l'extérieur depuis ledit revêtement (112) dans un plan perpendiculaire à un axe central s'étendant longitudinalement dudit revêtement (112), ledit joint torique externe (118) étant dans un état stable minimalement contraint lorsque lesdites surfaces chordales (118a, 118b) reposent dans ledit plan, ledit joint torique externe (118) pouvant être actionné, lorsqu'il est enroulé à 180 ° autour de son centroïde, pour enrouler ledit revêtement (112) sur lui-même et ainsi ajuster la longueur du revêtement (112) par incréments.

30. Dispositif chirurgical ajustable en longueur (110) selon la revendication 29, dans lequel au moins ledit anneau externe (118) est en uréthane ayant un duromètre dans une plage d'environ 50 à environ 90 Shore A.

31. Dispositif chirurgical ajustable en longueur (110) selon la revendication 29, dans lequel la largeur chordale de chaque surface chordale (118a, 118b) est définie sensiblement par la formule :

$$W = xD,$$

dans laquelle W est la largeur, x est une constante de 0,85, et D est la distance diamétrale entre lesdites surfaces arquées.

FIG. 1

22
14
16a
12
16
10

FIG. 2

12b
18
12b
12a
14
12
16
16a
14c
12b
20

FIG. 3

12
18a
12b
18
18b
12a
14a
14

FIG. 4

18a'
18'
18b'

FIG. 5A

FIG. 5B

EP 0 999 799 B1

FIG.6

FIG.7

FIG.8A

FIG.8B

17